(19) Europäisches Patentamt
European Patent Office
Office européen des brevets

(11) EP 1 668 102 B1

(12) **EUROPEAN PATENT SPECIFICATION**

(45) Date of publication and mention of the grant of the patent:
**31.10.2007 Bulletin 2007/44**

(21) Application number: **04761944.0**

(22) Date of filing: **29.09.2004**

(51) Int Cl.:
***C11B 9/00*** *(2006.01)* ***C07C 31/135*** *(2006.01)*
***C07C 49/297*** *(2006.01)*

(86) International application number:
**PCT/CH2004/000604**

(87) International publication number:
**WO 2005/030914 (07.04.2005 Gazette 2005/14)**

(54) **3-ISOPROPYL-1-METHYLCYCLOPENTYL DERIVATIVES AND THEIR USE IN FRAGRANCE APPLICATIONS**

3-ISOPROPYL-1-METHYLCYCLOPENTYLDERIVATE UND DEREN VERWENDUNG BEI RIECHSTOFFANWENDUNGEN

DERIVES DE 3-ISOPROPYL-1-METHYLCYCLOPENTYLE ET LEUR UTILISATION EN PARFUMERIE

(84) Designated Contracting States:
**AT BE BG CH CY CZ DE DK EE ES FI FR GB GR HU IE IT LI LU MC NL PL PT RO SE SI SK TR**

(30) Priority: **30.09.2003 GB 0322750**

(43) Date of publication of application:
**14.06.2006 Bulletin 2006/24**

(73) Proprietor: **Givaudan SA**
**1214 Vernier (CH)**

(72) Inventor: **BAJGROWICZ, Jerzy, A.**
**CH-8053 Zürich (CH)**

(74) Representative: **McStea, John Anthony**
**Givaudan Schweiz AG**
**Überlandstrasse 138**
**8600 Dübendorf (CH)**

(56) References cited:
**US-A- 4 234 463** **US-A- 4 533 492**

- **PATENT ABSTRACTS OF JAPAN vol. 0151, no. 45 (C-0823), 12 April 1991 (1991-04-12) & JP 3 024198 A (TAKASAGO INTERNATIONAL), 1 February 1991 (1991-02-01)**

EP 1 668 102 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

The present invention relates to 3-isopropyl-1-methylcyclopentyl derivatives, namely (3-isopropyl-1-methylcyclopentyl)ethanol, (3-isopropyl-1-methylcyclopentyl)ethanone and (3-isopropyl-1-methylcyclopentyl)methanol and their use as fragrances. This invention relates furthermore to a method for their production and to fragrance compositions comprising them.

In the fragrance industry there is a constant demand for new compounds that enhance or improve on odour notes, or impart new odour notes.

2-Alkylfenchyl alcohol derivatives are known from JP 3 024 198 A.

It has now been found that certain 3-isopropyl-1-methylcyclopentyl derivatives have much sought-after floral, fruity and woody odour notes, and they are relatively simple and easy to prepare starting from naturally available (1*S*)-(+)- and (1*R*)-(-)-fenchone.

Accordingly, the present invention refers in one of its aspects to the use of a compound of formula Ia and the enantiomer, namely (1*S*,3*R*)- enantiomer, thereof as fragrance

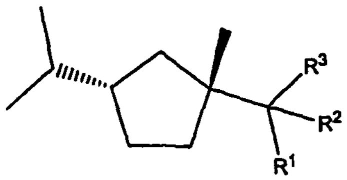

**Ia** **(1*R*, 3*S*)-**

wherein

$R^1$ is hydrogen or methyl;
$R^2$ is hydrogen; and
$R^3$ is hydroxyl; or
$R^2$ and $R^3$ form together with the carbon atom to which they are attached a carbonyl group.

It has been found that the odour threshold of certain compounds of formula Ia is on an average two times lower than that of the corresponding enantiomer. Accordingly, a compound of formula I

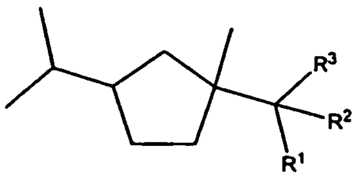

**I**

enriched in its (1*R*,3*S*) enantiomer of formula Ia are preferred.

In a further aspect, there is provided the use as fragrance of a compound of formula I

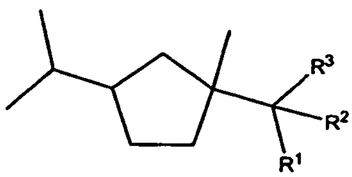

**I**

enriched in its (1*S*,3*R*) enantiomer of formula Ib

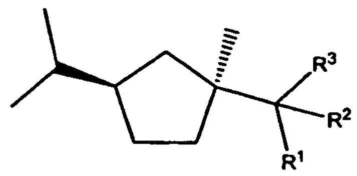

**Ib** **(1*S*,3*R*)-**

The term "enriched" is used herein to describe a compound having an enantiomeric purity greater than 1:1 in favour of the selected enantiomer. Compounds are preferred having a purity of about 1:3 or greater, e.g. 1:4. Particularly preferred are compounds having an enantiomeric purity of 1:9 or greater, such as 5:95 or 1:99.

Particularly preferred compounds of the present invention are [(1*R*,3*S*)-3-isopropyl-1-methylcyclopentyl]methanol, 1-[(1*R*,3*S*)-3-isopropyl-1-methylcyclopentyl]ethanone, and 1-[(1*R*,3*S*)-3-isopropyl-1-methylcyclopentyl]ethanol.

The compounds according to the present invention may be used alone or in combination with a base material. As used herein, the "base material" includes all known odourant molecules selected from the extensive range of natural products and synthetic molecules currently available, such as essential oils, alcohols, aldehydes and ketones, ethers and acetals, esters and lactones, macrocycles and heterocycles, and/or in admixture with one or more ingredients or excipients conventionally used in conjunction with odourants in fragrance compositions, for example, carrier materials, and other auxiliary agents commonly used in the art.

The following list comprises examples of known odourant molecules, which may be combined with the compounds of the present invention:

- ethereal oils and extracts, e.g. tree moss absolute, basil oil, castoreum, costus root oil, myrtle oil, oak moss absolute, geranium oil, jasmin absolute, patchouli oil, rose oil, sandalwood oil, wormwood oil, lavender oil or ylang-ylang oil;
- alcohols, e.g. citronellol, Ebanol™, eugenol, farnesol, geraniol, Super Muguet™, linalool, phenylethyl alcohol, Sandalore™, terpineol or Timberol™.
- aldehydes and ketones, e.g. α-amylcinnamaldehyde, Georgywood™, hydroxycitronellal, Iso E Super®, Isoraldeine®, Hedione®, maltol, Methyl cedryl ketone, methylionone or vanillin;
- ethers and acetals, e.g. Ambrox™, geranyl methyl ether, rose oxide or Spirambrene™.
- esters and lactones, e.g. benzyl acetate, Cedryl acetate, γ-decalactone, Helvetolide®, γ-undecalactone or Vetivenyl acetate.
- macrocycles, e.g. Ambrettolide, Ethylene brassylate or Exaltolide®.
- heterocycles, e.g. isobutylchinoline.

The compounds of the present invention may be used in a broad range of fragrance applications, e.g. in any field of fine and functional perfumery, such as perfumes, household products, laundry products, body care products and cosmetics. The compounds can be employed in widely varying amounts, depending upon the specific application and on the nature and quantity of other odourant ingredients. The proportion is typically from 0.001 to 20 weight percent of the application. In one embodiment, compounds of the present invention may be employed in a fabric softener in an amount of from 0.001 to 0.05 weight percent. In another embodiment, compounds of the present invention may be used in fine perfumery in amounts of from 0.1 to 20 weight percent, more preferably between 0.1 and 5 weight percent. However, these values are given only by way of example, since the experienced perfumer may also achieve effects or may create novel accords with lower or higher concentrations.

The compounds of the present invention may be employed into the fragrance application simply by directly mixing the fragrance composition with the fragrance application, or they may, in an earlier step be entrapped with an entrapment material, for example, polymers, capsules, microcapsules and nanocapsules, liposomes, film formers, absorbents such as carbon or zeolites, cyclic oligosaccharides and mixtures thereof, or they may be chemically bonded to substrates, which are adapted to release the fragrance molecule upon application of an external stimulus such as light, enzyme, or the like, and then mixed with the application.

Thus, the invention additionally provides a method of manufacturing a fragrance application, comprising the

incorporation of a compound of formula I enriched in one of their enantiomers, as a fragrance ingredient, either by directly admixing the compound to the application or by admixing a fragrance composition comprising a compound of formula I enriched in one of their enantiomers, which may then be mixed to a fragrance application, using conventional techniques and methods.

As used herein, "fragrance application" means any product, such as fine perfumery, e.g. perfume and eau de toilette; household products, e.g. detergents for dishwasher, surface cleaner; laundry products, e.g. softener, bleach, detergent; body care products, e.g. shampoo, shower gel; and cosmetics, e.g. deodorant, vanishing creme, comprising an odourant. This list of products is given by way of illustration and is not to be regarded as being in any way limiting.

Compared to most odorant molecules known in the art having floral odor properties, such as hydroxycitronellal, geranol, linalool and 4-(4-hydroxy-4-methylpentyl)cyclohex-3-ene-1-carbaldehyde, the compounds of formula Ia of the present invention, wherein $R^3$ is hydroxyl, are exceptionally stable both, under basic and acidic conditions, thus making them particula rly useful for a large variety of fragrance applications.

Compounds of formula Ia and the enantiomers thereof may be prepared by the Haller-Bauer rearrangement of (1*R*)-(-)-fenchone / (1*S*)-(+)-fenchone (1,3,3-trimethyl-2-norbornanone) followed by hydrolysis to 3-isopropyl-1-methylcyclopentanecarboxylic acid under alkali conditions, e.g. in the presence of a base such as NaOH or KOH. The resulting acid will then be reacted with methyllithium to give a compound of formula I wherein $R^2$ and $R^3$ form together with the carbon atom to which they are attached a carbonyl group. To give further compounds of the present invention, the resulting ketone may be transformed to a secondary alcohol through reduction, e.g. with $NaBH_4$.

(3-Isopropyl-1-methylcyclopentyl)methanol may be prepared by reduction of 3-isopropyl-1-methylcyclopentanecarboxylic acid (A), which has been prepared by rearrangement of fenchone, in the presence of LAH to the corresponding alcohol, as shown in scheme 1.

Scheme 1:

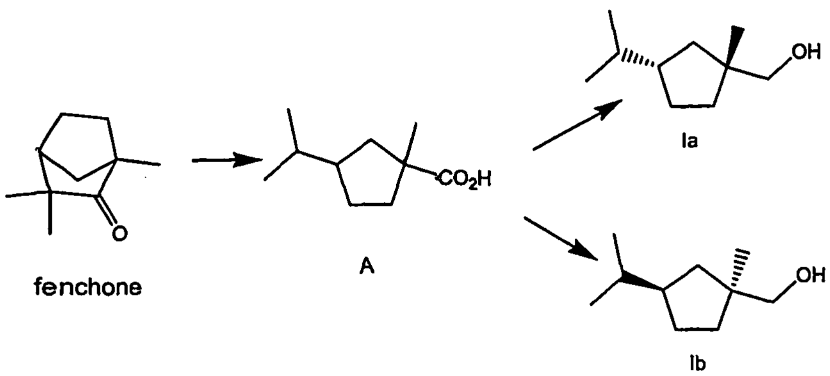

Optically pure compounds of formula Ia and Ib and enantiomeric mixtures of a compound of formula I enriched in one of the enantiomers, i.e. a compound of formula Ia or Ib, may be synthesised, starting from optically pure fenchone or an enantiomeric mixture enriched in either (1*R*)-(-)-fenchone or (1*S*)-(+)-fenchone.

The invention is now further described with reference to the following non-limiting examples.

All end products described in the following Examples 1 to 6 are colourless oils. They were obtained starting from (1*R*)-(-)- and (1*S*)-(+)-fenchone that contained 8% and 2% respectively of the other entantiomer. The reported NMR data were measured under the following general conditions: $^1H$ at 400 and $^{13}C$ at 100 MHz; in $CDCl_3$; chemical shifts (δ) in ppm downfield from TMS; coupling constants J in Hz.

Example 1: [(1*R*,3*S*)-3-Isopropyl-1-methylcyclopentyl]methanol

A solution of (1*R*,3*S*)-3-Isopropyl-1-methylcyclopentanecarboxylic acid (70.0 g, 0.41 mol), obtained from (1*R*)-(-)-fenchone (V. Braun, J.; Jacob, A. Chem. Ber. 1933, 66, 1461) in diethyl ether (100 ml) was slowly added, under nitrogen, to a suspension of lithium aluminium hydride (13.3 g, 0.35 mol) in the same solvent (500 ml). After heating at reflux during 3 h, the reaction mixture was cooled down to 10°C, 2N NaOH solution (70 ml) was carefully added and stirring continued for 0.5 h. The white solid was filtered off, the filtrate washed with brine (2 x 500 ml), dried ($MgSO_4$) and concentrated *in vacuo.* The crude product (79.0 g) was purified by distillation using a 10 cm Vigreux column (0.9-1.1 mbar, 96-98°C) to give [(1*R*,3*S*)-3-isopropyl-1-methylcyclopentyl]methanol (57.0 g, 90% yield).
$^1H$-NMR: δ 0.87 (*d, J* = 6.7, 3H), 0.88 (*d, J* = 6.7, 3H), 1.01 (*s*, 3H), 1.08 (*dd*, *J* = 12.3, 11.0, 1H), 1.16-1.38 (*m*, 3H),

1.48 (*ddd*, $J$ = 12.4, 6.9, 0.8, 1H), 1.53-1.72 (*m*, 3H), 1.74-1.87 (m, 1H), 3.35 (*d*, $J_{AB}$ = 10.4, 1H), 3.39 (*d*, $J_{AB}$ = 10.4, 1H). $^{13}$C-NMR: δ 21.5 (2q), 25.0 (q), 30.4 (t), 33.8 (d), 35.6 (t), 41.5 (t) 43.8 (s), 46.9 (d), 72.1 (t). $[\alpha]_D^{22}$ -12.0(*c* 1.0, EtOH).
Odour description: floral, green, jasmine, lily-of-the-valley, fresh, clean.

Example 2: [(1*S*,3*R*)-3-Isopropyl-1-methylcyclopentyl]methanol

Prepared according to the experimental procedure of Example 1 starting from (1*S*)-(+)-fenchone.
$[\alpha]_D^{22}$ +13.5 (*c* 1.0, EtOH).
Odour description: floral, fruity, green, rosy, hesperidic (grapefruit).

Example 3: 1-[(1*R*,3*S*)-3-Isopropyl-1-methylcyclopentyl]ethanone

A 1.6M solution of methyllithium in diethyl ether (200 ml, 0.32 mol) was added dropwise during 25 min. into a solution of (1*R*,3*S*)-3-Isopropyl-1-methylcyclopentanecarboxylic acid (25.5 g, 0.15 mol) in THF (250 ml) at 0°C. After stirring at 0°C for 3 h, chlorotrimethylsilane (151 ml, 1.2 mol) was added with cooling and the reaction mixture was allowed to warm up to room temperature, poured on ice-cold water (200 ml), stirred for 0.5 h and extracted with MTBE (2 x 250 ml). The combined organic phases were washed with water (200 ml), 2M NaOH (150 ml) and brine (3 x 200 ml), dried ($MgSO_4$) and concentrated in vacuo to give the crude 1-[(1*R*,3*S*)-3-isopropyl-1-methylcyclopentyl]ethanone (27.6 g), a sample of which (1.5 g) was purified by bulb-to-bulb distillation (0.93 g, 68% yield).
$^1$H-NMR: δ 0.89 (2*d*, $J$ = 6.6, 6H), 1.19 (s, 3H), 1.24 (*dq*, $J$ = 12.4, 9.1, 1H), 1.34-1.43 (m, 2H), 1.56-1.77 (m, 3H), 1.81-1.90 (m, 1H), 2.09 (*ddd*, $J$ = 13.1, 9.1, 4.0, 1H), 2.15 (s, 3H). $^{13}$C-NMR: δ 21.3 (q), 21.4 (q), 25.0 (q), 25.3 (q), 30.2 (t), 33.3(d), 35.6 (t), 41.0 (t), 46.6 (d), 55.3 (s), 213.0 (s). $\{\alpha]_D^{22}$ -1.0 (*c* 1.1, EtOH).
Odour description: earthy/mossy, green, woody.

Example 4: 1-[(1*S*,3*R*)-3-Isopropyl-1-methylcyclopentyl]ethanone

Prepared according to the experimental procedure of Example 3 starting from (1*S*)-(+)-fenchone.
$[\alpha]_D^{22}$ +1.0 (*c* 1.1, EtOH).
Odour description: floral, agrestic, fruity, green.

Example 5: 1-[(1*R*,3*S*) -3-Isopropyl-1-methylcyclopentyl]ethanol

A solution of 1-[(1*R*,3*S*)-3-isopropyl-1-methylcyclopentyl]ethanone from Example 3 (3.0 g, 18 mmol) in ethanol (8 ml) was added to a cold (ice-bath) solution of sodium borohydride (0.42 g, 10.7 mmol) in the same solvent (17 ml). After 1.5 h stirring at room temperature, the reaction mixture was poured on ice-cold 2M HCl (100 ml) and extracted with MTBE (2 x 100 ml). The combined organic phases were washed with brine (2 x 50 ml), dried ($MgSO_4$) and concentrated in vacuo. The crude product (2.8 g) was purified by bulb-to-bulb distillation (2.34 g, 77% yield, diastereoisomer ratio ~1:1).
$^1$H-NMR: δ 0.87 (*d*, $J$ = 6.6, 3H), 0.875 (*d*, $J$ = 6.6, 3H), 0.88 (2*d*, $J$ = 6.6, 6H), 0.92 (s, 3H), 0.93 (s, 3H), 1.05 (*t*, $J$ = 11.7, 1H), 1.12 (*d*, $J$ = 6.4, 3H), 1.125 (*d*, $J$ = 6.4, 3H), 1.14 (*t*, $J$ = 11.8, 1H), 1.17-1.74 (*m*, 12H), 1.47 (2s, 2H), 1.78-1.88 (m, 2H), 3.53 (*q*, $J$ = 6.3, 1H), 3.55 (q, $J$ = 6.3, 1H). $^{13}$C-NMR: δ 18.5 (2q), 21.3 (2q), 21.4 (3q), 21.5 (q), 29.8 (t), 30.0 (t), 33.7 (2d), 35.8 (t), 35.9 (t), 41.9 (2t), 46.3 (2d), 46.8 (s), 46.9 (s), 75.4 (d), 75.7 (d). $[\alpha]_D^{22}$ -7.0 (c 1.0, EtOH).
Odour description: floral, earthy/mossy, slightly terpineol/earthy.

Example 6: 1-[(1S,3*R*)-3-Isopropyl-1-methylcyclopentyl]ethanol

Prepared according to the experimental procedure of Example 5 starting from (1*S*)-(+)-fenchone.
$[\alpha]_D^{22}$ +8.0 (*c* 1.0, EtOH).
Odour description: hesperidic/citrus, fruity, green, fresh (grapefruit, rhubarb).

Example 7: Feminine Fine Fragrance

| Ingredient* | Parts per weight |
|---|---|
| Citronellol | 50 |
| Cyclamen aldehyde | 15 |
| Diethyl malonate | 5 |
| Dipropylene glycol (DPG) | 149 |
| Florhydral | 12 |
| Gardenol | 10 |
| Geraniol | 50 |
| Hedione | 25 |
| alpha-Hexylcinnamaldehyde | 200 |
| Hydroxycitronellal | 35 |
| Isocyclocitral 1% in DPG | 15 |
| Isojasmone | 2 |
| Jasmal | 40 |
| Jasmonyl | 20 |
| Lemon oil | 10 |
| Lilial | 25 |
| Linalool | 65 |
| Linalyl acetate | 50 |
| Methyl diantilis | 2 |
| Petitgrain Paraguay oil | 5 |
| Phenethyl alcohol | 65 |
| Silvial | 100 |
| [(1*R*,3*S*)-3-Isopropyl-1-methylcyclopentyl]methanol | 50 |
| | Total 1000 |

* for chemical names see Flavor and Fragrance Materials - 2003, Allured Publishing Corp. Carol Stream Ill., U.S.A..

The presence of 5% of [(1*R*,3*S*)-3-Isopropyl-1-methylcyclopentyl]methanol confers to this formula a creamy, lily-of-the-valley aspect.

Example 8: Floral Composition for Soap

| Ingredient* | Parts per weight |
|---|---|
| Agrumex | 100 |
| Benzophenone | 60 |
| Benzyl acetate | 55 |
| Bergamot base | 80 |
| 4-t-Butylcyclohexyl acetate | 150 |
| Diphenyl oxide | 20 |
| Dipropylene glycol (DPG) | 78 |
| Ebanol | 20 |
| Hydroxycitronellal | 200 |
| Jasmine base | 80 |
| Methyl Phenylacetate | 2 |
| Nerol | 20 |

(continued)

| Ingredient* | Parts per weight |
|---|---|
| Phenylpropyl alcohol | 40 |
| Rose base | 100 |
| Rhodinol | 65 |
| Sandela | 30 |
| Silvial | 100 |
| [(1*R*,3S)-3-Isopropyl-1-methylcyclopentyl]methanol | 50 |
| | Total 1250 |
| * for chemical names see Flavor and Fragrance Materials - 2003, Allured Publishing Corp. Carol Stream Ill., U.S.A.. | |

[(1*R*,3*S*)-3-Isopropyl-1-methylcyclopentyl]methanol makes this lily-of-the-valley fragrance velvety and rich.

## Claims

1. The use of a compound of formula Ia and the enantiomer thereof as fragrance,

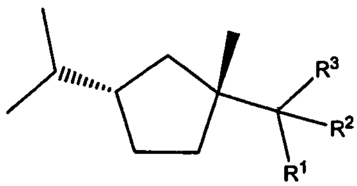

I a (1*R*,3*S*)-

wherein

$R^1$ is hydrogen or methyl;
$R^2$ is hydrogen; and
$R^3$ is hydroxyl; or
$R^2$ and $R^3$ form together with the carbon atom to which they are attached a carbonyl group.

2. The use as fragrance of a compound according to claim 1 selected from the group consisting of [(1*R*,3*S*)-3-isopropyl-1-methylcyclopentyl]methanol, [(1*S*,3*R*)-3-isopropyl-1-methylcyclopentyl]methanol, 1-[(1*R*,3*S*)-3-isopropyl-1-methylcyclopentyl]ethanone, 1-[(1*S*,3*R*)-3-isopropyl-1-methylcyclopentyl]ethanone, 1-[(1*R*,3*S*)-3-isopropyl-1-methylcyclopentyl]ethanol and 1-[(1*S*,3*R*)-3-isopropyl-1-methylcyclopentyl]ethanol.

3. The use as fragrance of a compound of formula I

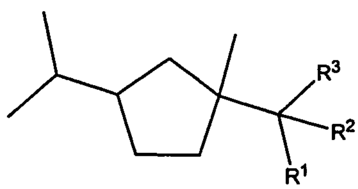

I

enriched in the enantiomer having the formula Ia

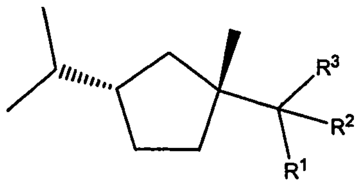

I a (1*R*,3*S* )-

wherein $R^1$, $R^2$ and $R^3$ have the same meaning as given in claim 1.

**4.** The use as fragrance of a compound of formula I

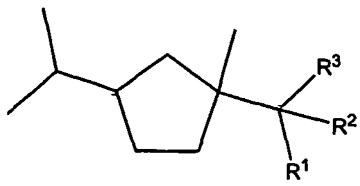

I

enriched in the enantiomer having the formula Ib

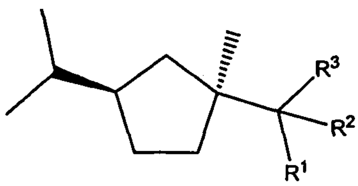

Ib (1*S*,3*R* )-

wherein $R^1$, $R^2$ and $R^3$ have the same meaning as given in claim 1.

**5.** The use of a compound as defined in one of the preceding claims in fragrance applications.

**6.** A fragrance application comprising a compound as defined in any of the preceding claims 1 - 4.

**7.** A fragrance application according to claim 6 wherein the fragrance application is a perfume, household product, laundry product, body care product or cosmetic products.

**8.** A method of manufacturing a fragrance application, comprising the step of incorporating a compound of formula Ia or its enantiomer as defined in claim 1, 2, 3 and 4.

**9.** A compound of formula Ia

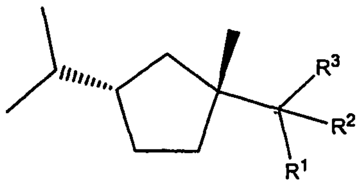

I a (1*R*,3*S*)-

wherein

$R^1$ is hydrogen or methyl;
$R^2$ is hydrogen; and
$R^3$ is hydroxyl; or
$R^2$ and $R^3$ form together with the carbon atom to which they are attached a carbonyl group.

**10.** A compound of formula Ib

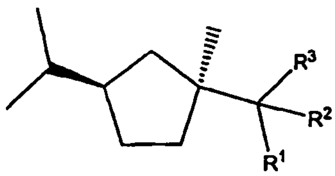

Ib (1*S*,3*R*)-

wherein

$R^1$ is methyl;
$R^2$ is hydrogen; and
$R^3$ is hydroxyl; or
$R^2$ and $R^3$ form together with the carbon atom to which they are attached a carbonyl group.

## Patentansprüche

**1.** Verwendung einer Verbindung der Formel Ia und des Enantiomers davon als Duft

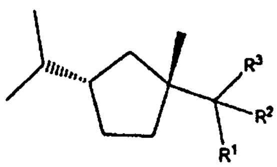

I a (1*R*,3*S*)- ,

wobei

$R^1$ für Wasserstoff oder Methyl steht;
$R^2$ für Wasserstoff steht und
$R^3$ für Hydroxyl steht oder
$R^2$ und $R^3$ zusammen mit dem Kohlenstoffatom, an das sie gebunden sind, eine Carbonylgruppe bilden.

**2.** Verwendung einer Verbindung nach Anspruch 1, die ausgewählt ist aus der Gruppe, bestehend aus [(1R,3S)-3-Isopropyl-1-methylcyclopentyl]methanol, [(1S,3R)-3-Isopropyl-1-methylcyclopentyl]methanol, 1-[(1R,3S)-3-Isopropyl-1-methylcyclopentyl]ethanon, 1-[(1S,3R)-3-Isopropyl-1-methylcyclopentyl]ethanon, 1-[(1R,3S)-3-Isopropyl-1-methylcyclopentyl]ethanol und 1-[(1S,3R)-3-Isopropyl-1-methylcyclopentyl]ethanol als Duft.

**3.** Verwendung einer Verbindung der Formel I

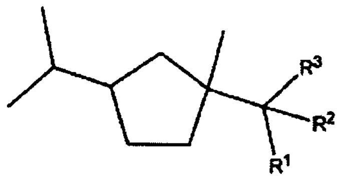

I

als Duft, die angereichert ist in dem Enantiomer, das die Formel Ia

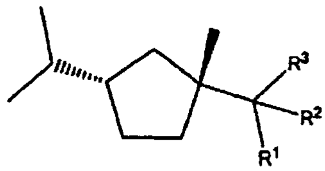

I a (1*R*,3*S*)-

aufweist, wobei $R^1$, $R^2$ und $R^3$ die gleichen Bedeutungen aufweisen, wie sie in Anspruch 1 angegeben sind.

**4.** Verwendung einer Verbindung der Formel I

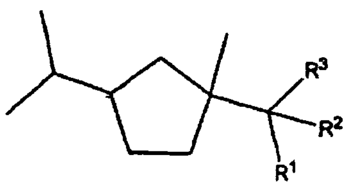

I

als Duft, die angereichert ist in dem Enantiomer, das die Formel Ib

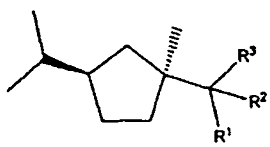

Ib (1*S*,3*R*)-

aufweist, wobei $R^1$, $R^2$ und $R^3$ die gleichen Bedeutungen aufweisen, wie sie in Anspruch 1 angegeben sind.

**5.** Verwendung einer Verbindung nach einem der vorstehenden Ansprüche in Duftanwendungen.

**6.** Duftanwendung, umfassend eine Verbindung wie in einem der vorstehenden Ansprüche 1 bis 4 definiert.

7. Duftanwendung nach Anspruch 6, wobei die Duftanwendung für ein Parfüm, Haushaltsprodukt, Wäscheprodukt, Körperpflegeprodukt oder kosmetische Produkte steht.

8. Verfahren zur Herstellung einer Duftanwendung, umfassend die Stufe eines Einverleibens einer Verbindung der Formel Ia oder seines Enantiomers wie in einem der Ansprüche 1, 2, 3 und 4 definiert.

9. Verbindung der Formel Ia

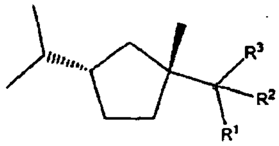

I a (1*R*,3*S*)-,

wobei

$R^1$ für Wasserstoff oder Methyl steht;
$R^2$ für Wasserstoff steht und
$R^3$ für Hydroxyl steht oder
$R^2$ und $R^3$ zusammen mit dem Kohlenstoffatom, an das sie gebunden sind, eine Carbonylgruppe bilden.

10. Verbindung der Formel Ib

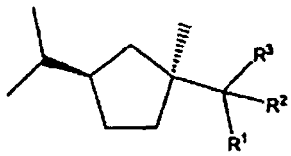

Ib (1*S*,3*R*)-,

wobei

$R^1$ für Methyl steht;
$R^2$ für Wasserstoff steht und
$R^3$ für Hydroxyl steht oder
$R^2$ und $R^3$ zusammen mit dem Kohlenstoffatom, an das sie gebunden sind, eine Carbonylgruppe bilden.

## Revendications

1. Utilisation d'un composé de formule Ia et de son énantiomère en tant que fragrance,

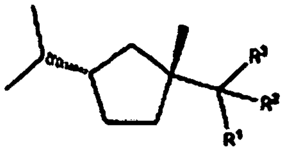

I a (1*R*,3*S*)-

dans laquelle

$R^1$ représente l'hydrogène ou un groupe méthyle ;
$R^2$ représente l'hydrogène ; et
$R^3$ représente un groupe hydroxyle ; ou
$R^2$ et $R^3$ forment conjointement avec l'atome de carbone auquel ils sont liés un groupe carbonyle.

**2.** Utilisation en tant que fragrance d'un composé selon la revendication 1, choisi dans le groupe constitué du méthanol de [(1R,3S)-3-isopropyl-1-méthylcyclopentyle], du méthanol de [(1S,3R)-3-isopropyl-1-méthylcyclopentyle], de l'éthanone de 1-[(1R,3S)-3-isopropyl-1-méthylcyclopentyle], de l'éthanone de 1-[(1S,3R)-3-isopropyl-1-méthylcyclopentyle], de l'éthanol de 1-[(1R,3S)-3-isopropyl-1-méthylcyclopentyle] et de l'éthanol de 1-[(1S,3R)-3-isopropyl-1-méthylcyclopentyle].

**3.** Utilisation en tant que fragrance d'un composé de formule I

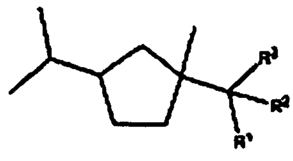

I

enrichi en l'énantiomère de formule Ia

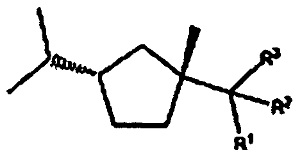

Ia (1R,3S)-

dans laquelle $R^1$, $R^2$ et $R^3$ sont tels que définis dans la revendication 1.

**4.** Utilisation en tant que fragrance d'un composé de formule I

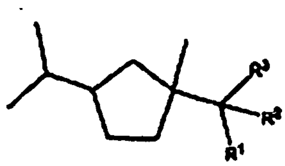

I

enrichi en l'énantiomère de formule Ib

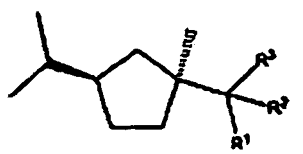

Ib (1S,3R)-

dans laquelle $R^1$, $R^2$ et $R^3$ sont tels que définis dans la revendication 1.

**5.** Utilisation d'un composé tel que défini selon l'une quelconque des revendications précédentes dans des applications de parfumerie.

**6.** Application de parfumerie comprenant un composé tel que défini dans l'une quelconque des revendications précédentes 1 à 4.

**7.** Application de parfumerie selon la revendication 6, dans laquelle l'application de parfumerie est un parfum, un produit d'entretien, un produit de lessive, un produit d'hygiène corporelle ou un produit cosmétique.

**8.** Procédé de préparation d'une application de fragrance, comprenant l'étape d'incorporation d'un composé de formule Ia ou de son énantiomère tel que défini selon les revendications 1, 2, 3 et 4.

**9.** Composé de formule Ia

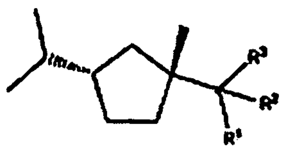

Ia (1R,3S)-

dans laquelle

$R^1$ représente l'hydrogène ou un groupe méthyle ;
$R^2$ représente l'hydrogène ; et
$R^3$ représente un groupe hydroxyle ; ou
$R^2$ et $R^3$ forment conjointement avec l'atome de carbone auquel ils sont liés un groupe carbonyle.

**10.** Composé de formule Ib

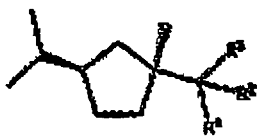

Ib (1S,3R)-

dans laquelle

$R^1$ représente un groupe méthyle ;
$R^2$ représente l'hydrogène ; et
$R^3$ représente un groupe hydroxyle ; ou
$R^2$ et $R^3$ forment conjointement avec l'atome de carbone auquel ils sont liés un groupe carbonyle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3024198 A


### Non-patent literature cited in the description


- **V. BRAUN, J. ; JACOB, A.** *Chem. Ber.,* 1933, vol. 66, 1461
- Flavor and Fragrance Materials. Allured Publishing Corp. Carol Stream III, 2003
- Flavor and Fragrance Materials. Allured Publishing Corp. Carol Stream III, 2003